(19) Europäisches Patentamt
European Patent Office
Office européen des brevets

(11) Publication number: **0 260 796 B1**

(12) # EUROPEAN PATENT SPECIFICATION

(45) Date of publication of patent specification: **17.04.91**

(51) Int. Cl.⁵: **A61K 37/02**, A61K 31/785, A61K 47/00, C07K 15/00, A61K 9/10

(21) Application number: **87306751.6**

(22) Date of filing: **30.07.87**

The file contains technical information submitted after the application was filed and not included in this specification

(54) **Compositions of neocarzinostatin derivative for oral administration.**

(30) Priority: **19.09.86 JP 221166/86**

(43) Date of publication of application: **23.03.88 Bulletin 88/12**

(45) Publication of the grant of the patent: **17.04.91 Bulletin 91/16**

(84) Designated Contracting States: **AT BE CH DE FR GB IT LI NL SE**

(56) References cited:
**EP-A- 0 087 957**
**EP-A- 0 136 791**
**EP-A- 0 136 792**
**GB-A- 2 114 885**
**US-A- 4 182 752**

**CHEMICAL ABSTRACTS, vol. 91, no. 20, November 12, 1979, Columbus, Ohio, USA**

**TAKESHITA et al. "A lipophilic derivative of neocarzinostatin. A polymer conjugation of an antitumor protein antibiotic" page 345, column 1, abstract-no. 162 995j & Int. J.Pept. Protein Res., vol. 14, no. 2, 1979, pages 81-87**

(73) Proprietor: **YAMANOUCHI PHARMACEUTICAL CO. LTD.**
**No. 3-11 Nihonbashi-Honcho, 2-chome Chuo-ku**
**Tokyo(JP)**

Proprietor: **Maeda, Hiroshi**
**631-3, Aza-Tamukae Hotakubohon-Machi**
**Kumamoto City Kumamoto Pref.(JP)**

Proprietor: **KURARAY CO., LTD.**
**1621 Sakazu**
**Kurashiki-City Okayama Prefecture 710(JP)**

Proprietor: **KAYAKU ANTIBIOTICS RESEARCH CO., LTD.**
**8-16, Funado 2-Chome**
**Itabashi-Ku Tokyo(JP)**

(72) Inventor: **Maeda, Hiroshi**
**631-3, Aza-Tamukae Hotakubonon-Machi**
**Kumamoto City Kumamoto Pref(JP)**
Inventor: **Suzuki, Fujio**
**573-20, Shimonabe-Machi**
**Kumamoto City Kumamoto Pref(JP)**

**EP 0 260 796 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: Oka, Kiichiro
403, Hosei Bldg., 7-28 Honjo 3-chome
Kumamoto City Kumamoto Pref(JP)
Inventor: Tanaka, Shohei
515, Yotsugugu Bldg 1-8, Honjo 3-chome
Kumamoto City Kumamoto Pref(JP)

(74) Representative: Myerscough, Philip Boyd et al
J.A.Kemp & Co. 14, South Square Gray's Inn
London, WC1R 5EU(GB)

## Description

This invention relates to a composition for oral administration comprised of a neocarzinostatin derivative (hereinafter abbreviated as SMANCS) having styrene-maleic acid copolymeric residue.

SMANCS is a derivative of neocarzinostatin (hereinafter abbreviated as NCS) as a proteinaceous anticancer substance, wherein carbonyl groups of styrenemaleic acid copolymer (hereinafter abbreviated as SMA) are bonded to two primary amino groups existent in the molecule of NCS through acid amide (see EP-A-136791). That is, it is a cancerocidal substance having a reduced toxicity and improved pharmacological properties as compared with those of NCS.

It has been considered that SMANCS cannot orally be administered because it is decomposed gastrically and intestinally by digestive juices as are other high molecular weight biogenic peptides and proteins, and has poor absorbability. Thus, SMANCS is generally administered by injection. In this case, however, pain is unavoidable to patients and also self-administration is impossible. Furthermore, for daily injections patients need to be hospitalized, and this situation results in more expense. Therefore, it is desired to develop the preparation of SMANCS composition for oral administration.

It has been attempted to provide biogenic peptides for oral administration (for instance, JP-B-61-93,129 and EP-A-177342 disclose a preparation for oral administration comprising a lipotropic medium such as mineral oil and a gastrocolic absorption accelerator such as sodium salicylate). Since, however, SMANCS is obtained by bonding SMA as a polymer to NCS as a high molecular weight biogenic peptide, it is a very special cancerocidal substance possessing properties both as a peptide and as a polymer, so that a preparation for oral administration attempted for a simpler biogenic peptide cannot be applied to SMANCS. Indeed, we tried to prepare SMANCS with an oily substance such as a fatty acid, fatty acid alkyl ester, cholesterol or mineral oil for oral administration, but were unsuccessful.

Antitumor drug compositions comprising a water soluble or sparingly oil soluble antitumor drug, a fat or oil vehicle and a solubiliser are described in GB -A-2114885 but are intended for administration into organ-feeding blood vessels and/or lymphatics.

This invention provides a composition for oral administration comprising SMANCS and at least one medium to long chain fatty acid glyceride which is a mono-, di- or tri-glyceride of a saturated or unsaturated fatty acid having a carbon number of 6 to 20. The invention also provides a composition for oral administration comprising SMANCS, at least one said medium to long chain fatty acid glyceride and an amphiphilic agent.

Here, SMANCS is a neocarzinostatin derivative represented by the formula:

(SMA)-(NCS)-(SMA)

wherein (NCS) is a divalent neocarzinostatin residue in which one hydrogen atom is removed from the primary amino group in (a) the alanine residue at the N-terminal of neocarzinostatin and (b) the lysine residue at the 20th position from the N-terminal of neocarzinostatin and (SMA) is a monovalent styrene-maleic acid copolymeric residue, which may be partially half-esterified and consists of structural units of the formulae 1) a styrene residue of formula

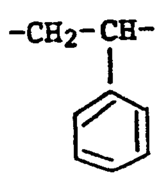

2) a maleic acid residue of formula

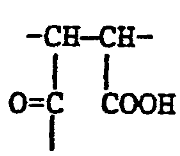

wherein the carbon atom of the carbonyl group is bonded to the neocarzinostatin residue; and 3), a) maleic

acid residue of formula

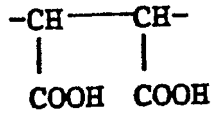

and optionally b) a half-esterified maleic acid residue of formula

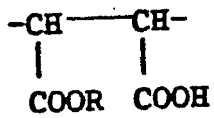

wherein R is a residue of an alcohol ROH which is an alkanol having 1 to 4 carbon atoms, an ethylene glycol monoalkyl ether in which the alkyl group has 1 or 2 carbon atoms or a glycerine dialkyl ether wherein the alkyl group has 1 or 2 carbon atoms.

The invention will be described with reference to the accompanying drawings, wherein:

Fig. 1 is a graph showing a change of Bu-SMANCS concentration in blood with time after oral administration of the composition according to the invention; and

Fig. 2 is a graph showing a change of intracorporeal distribution of $^{14}C$ glycine labelled Bu-SMANCS with time after oral administration.

Details of SMANCS are disclosed in EP-A-136791 and EP-A-136792 and a typical example is a compound wherein the styrene-maleic acid copolymeric residue as (SMA) is a half-butyl esterified styrene-maleic acid copolymeric residue (hereinafter abbreviated as Bu-SMANCS).

The medium to long chain fatty acid glyceride is mono-, di- or tri-glyceride of a saturated or unsaturated fatty acid having a carbon number of 6 to 20. As the fatty acid glyceride, mention may be made of mono-, di- and tri-glycerides of caprylic acid, capric acid, lauric acid, myristic acid, palmitic acid, oleic acid, linoleic acid and linolenic acid. These fatty acid glycerides may be used alone or in admixture. Further, the fatty acid glyceride may be natural, synthetic or semi-synthetic substance. In general, it is convenient to use natural vegetable oils. As the preferred vegetable oil used in the invention, mention may be made of olive oil (oleic acid: 70~85%, linoleic acid: 4~12%, palmitic acid: 7~15%), maize oil (linoleic acid: 40~60%, palmitic acid: 25~45%), sesame oil (oleic acid: 35~46%, linoleic acid: 35~48%), tsubaki oil, coconut oil (lauric acid: 45~52%, capric acid: 4~12%, caprylic acid: 6~10%), palm oil and so on. Moreover, commercially available vegetable oils may be used as they are. The commercially available medium chain fatty acid triglyceride includes Panasate 875®, Panasate 810® and Panasate 800® (registered trade name) made by Nippon Oil and Fats Co., Ltd. (content of caprilic acid: 10~100%), ODO® (registered trade name) made by Nisshin Seiyu K.K. (content of caprilic acid: 67%) and the like. The medium chain fatty acid monoglyceride includes Homoteks PT® (registered trade name) made by Kao Corporation (content of capric acid: about 60%) and so on. As a mixture of medium chain fatty acid monoglyceride and diglyceride, there are Witafrol® (registered trade name) made by Dinamit Nobel Corporation and so on. As the long chain fatty acid triglyceride, use may be made of commercially available olive oil, safflower oil and other edible oils.

The amphiphilic agent is a non-toxic substance possessing hydrophilicity and lipophilicity, which includes, for example, natural ampholytic surfactant, polyglycerine fatty acid ester, polyoxyethylenesorbitan fatty acid ester (Tween series), sorbitan fatty acid ester (Span series), polyethylene glycol and so on.

As the natural ampholytic surfactant, use may be made of soybean phospholipid, yolk lecithin and analogous compounds such as phosphatidylcholine made by Nippon Oil and Fats Co., Ltd., yolk lecithin, soybean lecithin, phosphatidylethanolamine and so on. Further, Unigli® (registered trade name) made by Nippon Oil and Fats Co., Ltd. may be used as a polyglycerine fatty acid ester, and Tween 20® (registered trade name, sold by Wako Junyaku Kogyo K.K.) may be used as a polyoxyethylenesorbitan fatty acid ester, and Span 20® (registered trade name, sold by Wako Junyaku Kogyo K.K.) may be used as a sorbitan fatty acid ester, and PEG 6000 (trade name) may be used as a polyethylene glycol. Besides this, sodium lauryl sulfate may be used as an anion surfactant, and benzalkonium chloride and benzethonium chloride, Azone® (registered trade name, made by Nelson Res. & Dev. Co.) may be used as a cation surfactant.

In the preparation of the composition according to the invention, a lyophilized powder of SMANCS is

added to the medium to long chain fatty acid glyceride and uniformly dispersed thereinto. When using the amphiphilic agent, it may be added at any stage. That is, SMANCS and the amphiphilic agent may be mixed in water and then lyophilized in the production of the SMANCS lyophilized powder, or the amphiphilic agent may be added to the medium to long chain fatty acid glyceride before the mixing with the SMANCS lyophilized powder. This is applicable to a case of using two or more amphiphilic agents.

An example of preparation of the composition according to the invention is as follows:

(a) The lyophilized powder of SMANCS is added to the medium to long chain fatty acid glyceride previously mixed or not mixed with the amphiphilic agent and then uniformly dispersed thereinto to form an oily composition. Moreover, the lyophilized powder of SMANCS as a starting material is obtained by lyophilizing an aqueous solution of SMANCS in a usual manner.

(b) SMANCS and amphiphilic agent are mixed in water. Preferably, the aqueous solution of SMANCS is mixed with the aqueous solution of amphiphilic agent at equal weight. Then, the resulting mixture is lyophilized and added to the medium to long chain fatty acid glyceride previously mixed or not mixed with the amphiphilic agent and uniformly dispersed thereinto to form an oily composition.

In the uniform dispersion, the shake-agitation is continued till the sufficient dispersed state is visually confirmed. For instance, the shake-agitation is carried out by an ultrasonic treatment using an ultrasonic generator. Moreover, in order to enhance the uniform dispersibility in the composition according to the invention, a lower alcohol such as ethanol or the like may previously be added to the medium to long chain fatty acid glyceride.

The above procedures are carried out at room temperature or under cooling, e.g. in an ice-water bath, whereby the composition according to the invention can be prepared.

According to the invention, the amount of the fatty acid glyceride used is about 0.l~l00 mℓ, preferably 0.5~5 mℓ per l mg of SMANCS. The addition of the amphiphilic agent is not necessarily required, but it provides a good oil-wetting effect to increase the dispersion solubility and hence form a stable composition, and brings about the absorption promoting effect. The amount of the amphiphilic agent added is changed in accordance with the kind thereof, but it is usually 0.0l~0.l mℓ in case of a liquid agent and 0.05~5 mg in case of a solid agent per l mg of SMANCS. Moreover, the amount of the lower alcohol added for enhancing the dispersibility is 0.0l~0.5 mℓ, preferably 0.05~0.2 mℓ per l mg of SMANCS.

The oily composition according to the invention is stable physically and chemically. That is, sediments are not observed even when the composition is subjected to a centrifugal separation at room temperature and 5,000 rpm for l5 minutes or is left to stand at 37° C for one month. Further, the anticancer activity of SMANCS is not reduced even when the composition is stored at 4° C or room temperature under shading for at least 3 months. Moreover, even when the composition is subjected to temperature change of 4° C and room temperature every 24 hours ten times, no change is visually observed and the titer is stable.

As mentioned above, the composition according to the invention has excellent stability and makes possible to administer SMANCS orally, whereby the utility value of SMANCS as an anticancer agent is more enhanced.

The effect of the composition according to the invention will concretely be described by the following animal experiments.

(l) Test for anticancer activity:

The anticancer activities against RL ♂ l murine solid tumor, S-l80 murine solid tumor, S-l80 murine ascites-type tumor and Meth A murine solid tumor are set forth with their experimental methods.

(l) Inhibition effect against RL ♂ l murine solid tumor

Experimental method:

After $l0^6$ RL ♂ l tumor cells were subcutaneously transplanted into a ventral side portion of BALB/c mouse (8 week age), a medication to be tested as mentioned later was administered to the mouse one time per day from next day of the transplantation over a week. After 2l days of the tumor transplantation, the mouse was killed to measure the tumor weight.

The measured results are shown in the following Table l.

Table 1

| Test medicine | Dose | | Administration pathway | Tumor weight (mg) | Inhibition ratio (%) |
|---|---|---|---|---|---|
| | (mℓ/mouse) | (mg/Kg) as Bu-SMANCS | | | |
| Physiological saline (control) | 0.2 | | oral | 2,029 | - |
| ODO® alone (control) | 0.2 | | oral | 2,034 | 0 |
| Bu-SMANCS* (control) | 0.1 | 0.1 | intravenous injection | 303 | 85 |
| Composition of Preparation Example 11 (drug concentration: 1.5 mg/mℓ) | 0.2 | 10.1 | oral | 816 | 60 |
| Composition of Preparation Example 11 (drug concentration: 0.15 mg/mℓ) | 0.2 | 1.0 | oral | 1,197 | 41 |

* Positive control for aqueous intravenous injection (drug concentration: 0.03 mg/mℓ)

(2) Inhibition effect against S-180 murine solid tumor

Experimental method:

After $10^6$ S-180 tumor cells were subcutaneously transplanted into a ventral side portion of ddY mouse (1 group: 10 mice), a medication to be tested was administered to the mouse one time per day from next day of the transplantation over a week. In 17 days after the transplantation, the tumor portion was extirpated to measure the weight.

The measured results are shown in the following Table 2.

Table 2

| Test medicine | Dose | | Administration pathway | Tumor weight (mg) | Inhibition ratio (%) |
|---|---|---|---|---|---|
| | (mℓ/mouse) | (mg/Kg) as Bu-SMANCS | | | |
| Physiological saline (control) | 0.2 | | oral | 776 | - |
| ODO® alone (control) | 0.2 | - | oral | 744 | 4 |
| Bu-SMANCS* (control) | 0.1 | 0.1 | intravenous injection | 64 | 92 |
| Bu-SMANCS** (control) | 0.2 | 10.1 | oral | 795 | 0 |
| Composition of Preparation Example 11 (drug concentration: 1.11 mg/mℓ) | 0.2 | 1.0 | oral | 107 | 86 |

* Positive control for aqueous intravenous injection (drug concentration: 0.01 mg/mℓ)

** Positive control for aqueous intravenous injection (drug concentration: 1.5 mg/mℓ)

(3) Inhibition effect against S-180 murine ascites-type tumor

Experimental method:

After $10^6$ S-180 tumor cells were intraabdominally transplanted into ddY mouse (8 week age), a medication to be tested was administered to the mouse one time per day from next day of the transplantation over a week. In 12 days after the transplantation, the mice were killed to measure the tumor weight.

The measured results are shown in the following Table 3.

Table 3

| Test medicine | Dose | | Administration pathway | Tumor weight (mg) | Inhibition ratio (%) |
|---|---|---|---|---|---|
| | (mℓ/mouse) | (mg/Kg) as Bu-SMANCS | | | |
| Physiological saline (control) | 0.2 | | oral | 1,260 | - |
| ODO® alone (control) | 0.2 | | oral | 1,348 | 0 |
| Bu-SMANCS* (control) | 0.1 | 0.5 | intravenous injection | 911 | 28 |
| Composition of Preparation Example 11 (drug concentration: 1.11 mg/mℓ) | 0.2 | 7.4 | oral | 951 | 25 |
| Composition of Preparation Example 11 (drug concentration: 0.375 mg/mℓ) | 0.2 | 2.5 | oral | 1,001 | 21 |

* Positive control for aqueous intravenous injection (drug concentration: 0.15 mg/mℓ)

(4) Inhibition effect against Meth A murine solid tumor

After $10^6$ Meth A murine tumor cells were subcutaneously transplanted into a ventral side portion of BALB/c mouse (8 week age), a medication to be tested was administered to the mouse one time per day from next day of transplantation over a week. In 14 days after transplantation, the mice were killed to measure the tumor weight.

The measured results are shown in the following Table 4.

Table 4

| Test medicine | Dose | | Admini-stration pathway | Tumor weight (mg) | Inhi-bition ratio (%) |
|---|---|---|---|---|---|
| | (mℓ/mouse) | (mg/Kg) as Bu-SMANCS | | | |
| Physiological saline (control) | 0.2 | | oral | 240 | - |
| ODO® alone (control) | 0.2 | | oral | 235 | 2 |
| Bu-SMANCS* (control) | 0.1 | 0.1 | intra-venous injection | 6 | 98 |
| Composition of Preparation Example 11 (drug concentration: 1.5 mg/mℓ) | 0.2 | 10.0 | oral | 27 | 89 |
| Composition of Preparation Example 11 (drug concentration: 0.15 mg/mℓ) | 0.2 | 1.0 | oral | 137 | 43 |

* Positive control for aqueous intravenous injection (drug concentration: 0.03 mg/mℓ)

(II) Transport of oily Bu-SMANCS into the blood after the oral administration:

The concentration of Bu-SMANCS in the blood was measured by bioassay or radioactivity analysis. The following results were obtained.

(I) Measurement of bioactivity

(a) Test method and results using bacterium

To a mouse (ddY, 8 week age) was orally and forcedly administered 1.0 mℓ of the composition in Preparation Example 5 as mentioned later (containing 4 mg/mℓ of oily Bu-SMANCS) through a stainless stomach tube. After the administration, blood (or plasma) was taken out every given time and then the bioactivity of Bu-SMANCS in the blood was measured by using Micrococcus luteus according to a method described by H. Maeda, J. Takeshita and A. Yamashita, Eur. J. Cancer, 16 , pp 723-731, 1980. At 3 hours after the administration, the bioactivity in blood became maximum, and the drug concentration in blood equal to that through intravenous injection was obtained. From this fact, it is considered that the orally administered oily Bu-SMANCS is easily absorbed by a bowel a nd distributed via the general circulation and lymphatic system in the whole body. The concentration of Bu-SMANCS in plasma after 3 hours of administration is shown in the following Table 5.

Table 5

| Time after administration | Drug concentration in plasma * |
|---|---|
| 3 hours | 2.5 μg/mℓ |

*: based on bioactivity, use of original Bu-SMANCS as standard

(b) Test method and results using culture cell

To a mouse (ddY, 8 week age) fasted over more than l2 hours was orally and forcedly administered l.0 mℓ of a composition in Preparation Example 28 as mentioned later (containing 4 mg/mℓ of Bu-SMANCS) or l.0 mℓ of an aqueous solution of Bu-SMANCS in physiological saline (containing 4 mg/mℓ of Bu-SMANCS) as an aqueous intravenous injection positive control (PBS: phosphate buffered 0.l5M saline) through a stainless stomach tube. After the administration, 30 μℓ of blood was drawn from a tail vena of the mouse at 0, l, 3, 5, 7, 9 or l2 hours and added to 300 μℓ of a culture medium containing culture cells. The culture cells were prepared by diluting EB virus-transformed B lymophoblastomid cells at logarithmic growth phase in the culture medium at a concentration of $2\times l0^5$ cells/mℓ and pouring the resulting dilution into each of 96 holes in a plastic plate (Falcon) in an amount of 300 μℓ. After the addition of blood, the plate was cultured at 37° C in an atmosphere containing 5% of $CO_2$ and 95% air. After two days of culture, they were stained with trypan blue and the number of living cells were counted, from which a survival rate was calculated. Then, the concentration of Bu-SMANCS in the blood was measured from the standard curve. The results are shown in Fig. l, wherein mark ● shows results using the composition according to the invention and mark ○ shows results using SMANCS in aqueous (PBS) form administered orally as control. Two mice for each point were used.

As seen from Fig. l, the composition according to the invention gave prolonged retention time of Bu-SMANCS in blood through oral administration as compared with the aqueous (PBS) oral administration as control. The oily formulation according to the invention gave much higher plasma concentration. Furthermore, the area under curve of the concentration in the plasma is increased greatly to ll fold.

(2) Activation analysis

(i) Preparation of $^{14}C$ glycine labelled Bu-SMANCS

l8.3 mg of Bu-SMANCS was dissolved in l.0 g of distilled water, to which was added l9.9 mg of water-soluble carbodiimide and after 30 minutes, 0.l mg of l4C glycine (l.5 mℓ aqueous solution: New England Nuclear Corp., ll3.0 m Ci/m moℓ) was added. The resulting solution was adjusted with an aqueous solution of lM $NaHCO_3$ so as to make pH about 6 and reacted at room temperature for l hour with stirring. After the reaction was stopped by adding l.0 mℓ of lM acetic acid buffer solution (pH = 6.0), the reaction product was desalted by a Sephadex® G-25 column (2.3×l0 cm) and then lyophilized to obtain a fluffy sample (l6.7 mg of $^{14}C$ glycine labelled Bu-SMANCS). It gave a specific radioactivity of l.27 μ Ci/mg, wherein one glycine was mainly introduced into one molecule of Bu-SMANCS. This single sample is a stable compound wherein glycine is bonded to carboxyl group of Bu-SMANCS through amide bond.

(ii) Animal experiment through oral administration

A composition of Preparation Example 2 as mentioned later was prepared by using the above $^{14}C$

glycine labelled Bu-SMANCS. Then, 0.l mℓ (0.64 μ Ci) of this composition was forcedly and orally administered to a mouse (ddY♂, 8 week age) through a stainless stomach tube. After the administration, the mouse was killed at l, 3, 7 or 24 hours to measure the radioactivity in plasma and major organs. The measured results are shown in Fig. 2, wherein mark Δ is the liver; O, the blood plasma; □, the kidney and ◊, the stomach. Moreover, the radioactivity is represented by dpm per l.0 g of tissue. Fig. 2 shows that the composition according to the invention is efficiently transferred into the blood and each intracorporeal tissues likewise the aforementioned data of bioassay.

As mentioned above, the composition according to the invention exhibited an excellent anticancer effect through the oral administration.

As a clinical application to human, the composition according to the invention is prepared into a appropriate formulation such as capsule, soft capsule, tablet, granule, or liquid, which is administered to a cancer patient by oral administration.

The administration is carried out every day or every other day l to 5 times per day in such a manner that a dose per time of SMANCS is 0.l~l00 mg. The composition according to the invention can be applied to solid tumor as well as liquid tumor (leukemia) and ascitic tumor.

The preparation of the composition according to the invention will be explained with following examples. In these examples, Bu-SMANCS is used as SMANCS, but similar results may be obtained by using the other SMANCS.

Preparation Method l

To the lyophilized powder of Bu-SMANCS was added the medium to long chain fatty acid glycerides previously mixed with the amphiphilic agent, and the resulting mixture was shaken till dispersed completely as confirmed visually. In this case, the ultrasonic treatment was carried out by using a chip-type ultrasonic generator, Model UR-l50P: Tomy Seiki K.K. The time required was no more than 30 seconds.

Preparation Method 2

40 mg of the amphiphilic agent was dissolved in 5 mℓ of distilled water by the ultrasonic treatment, and the aqueous solution of l% ammonium carbonate was added dropwise to adjust pH to about 8. The resulting solution was mixed with a solution of Bu-SMANCS powder (4 mg/mℓ) dissolved in aqueous solution of 0.02% ammonium carbonate under ice cooling at an equal weight ratio under stirring, which was then lyophilized. The resulting lyophilized powder was added to the medium to long chain fatty acid glyceride and subjected to an ultrasonic treatment in ice for 30 seconds.

The composition according to the invention having a composit ratio shown in the Table 6 was obtained by the above preparation method l or 2.

Table 6(a)

| Preparation Example No. | Bu-SMANCS | Medium to long chain fatty acid glyceride | Amphiphilic agent | Preparation method |
|---|---|---|---|---|
| 1 | 1 mg | Panasate 875® 1 mℓ | phosphatidylcholine 0.1 g | 1 |
| 2 | 1 mg | Panasate 810® 0.95 mℓ | Unigli GO-206® 0.05 mℓ | 2 |
| 3 | 1 mg | Panasate 800® 0.25 mℓ<br>olive oil 0.75 mℓ | yolk lecithin 0.1 g | 1 |
| 4 | 1 mg | O.D.O.® 0.25 mℓ<br>safflower oil 0.75 mℓ | soybean lecithin 0.1 g | 1 |
| 5 | 1 mg | Panasate 875® 0.95 mℓ | phosphatidylethanolamine 2 mg<br>Unigli GO-206® 0.05 mℓ | 2 |
| 6 | 1 mg | Panasate 875® 0.8 mℓ<br>Homoteks PT® 0.2 mℓ | phosphatidylcholine 2 mg | 2 |
| 7 | 1 mg | O.D.O® containing<br>10% ethanol 1 mℓ | yolk lecithin 2 mg | 2 |
| 8 | 1 mg | Panasate 810® 0.95 mℓ | phosphatidylcholine 0.1 g<br>Unigli GO-206® 0.05 mℓ | 2 |
| 9 | 1 mg | O.D.O.® 1 mℓ | soybean lecithin 0.1 g<br>PEG6000 0.1 g | 2 |
| 10 | 1 mg | Panasate 800® 0.9 mℓ | Tween 20® 0.05 mℓ<br>Span 20® 0.05 mℓ | 2 |
| 11 | 1 mg | O.D.O.® 1 mℓ | ——— | 1 |
| 12 | 1 mg | Panasate 800® 1 mℓ | ——— | 1 |
| 13 | 1 mg | Panasate 810® 1 mℓ | ——— | 1 |
| 14 | 1 mg | safflower oil 1 mℓ | ——— | 1 |
| 15 | 1 mg | olive oil 1 mℓ | ——— | 1 |

Table 6(b)

| Preparation Example No. | Bu-SMANCS | Medium to long chain fatty acid glyceride | Amphiphilic agent | Preparation method |
|---|---|---|---|---|
| 16 | 1 mg | Panasate 875® 1 mℓ | ——— | 1 |
| 17 | 1 mg | edible oil 1 mℓ (made by Nisshin Seiyu K.K.) | ——— | 1 |
| 18 | 1 mg | O.D.O.® 0.5 mℓ<br>edible oil 0.5 mℓ (made by Nisshin Seiyu K.K.) | ——— | 1 |
| 19 | 1 mg | O.D.O.® 0.5 mℓ<br>edible oil 0.5 mℓ (made by Nisshin Seiyu K.K.) | phosphatidylcholine 1 mg | 2 |
| 20 | 1 mg | O.D.O.® 0.95 mℓ | Azone® 0.05 mℓ | 2 |
| 21 | 0.1 mg | O.D.O.® 1 mℓ | ——— | 1 |
| 22 | 10 mg | O.D.O.® 1 mℓ | ——— | 1 |
| 23 | 20 mg | O.D.O.® 1 mℓ | ——— | 1 |
| 24 | 30 mg | O.D.O.® 1 mℓ | ——— | 1 |
| 25 | 1 mg | Panasate 875® containing 10% ethanol 0.95 mℓ | phosphatidylethanolamine 2 mg<br>Unigli GO-206® 0.05 mℓ | 2 |
| 26 | 1 mg | Panasate 875® containing 5% ethanol 0.8 mℓ<br>Homoteks PT® 0.2 mℓ | phosphatidylcholine 2 mg | 2 |
| 27 | 1 mg | Panasate 810® containing 15% ethanol 0.95 mℓ | phosphatidylethanolamine 0.1 g<br>Unigli GO-206® 0.05 mℓ | 2 |

Preparation Example 28

4 mg of Bu-SMANCS and 8mg of phosphatidylcholine as an amphiphilic agent were mixed in water and then lyophilized. The resulting lyophilized powder was added to l mℓ of Panasate 875® (medium chain fatty acid glyceride) containing 0.05 mℓ of Unigli GO-206® (amphiphilic agent, polyglycerine (6) dioleate) and uniformly dispersed thereinto by the ultrasonic treatment in ice.

## Claims

**1.** A composition suitable for oral administration comprising a neocarzinostatin derivative , and at least one medium to long chain fatty acid glyceride which is a mono-, di- or tri-glyceride of a saturated or unsaturated fatty acid having a carbon number of 6 to 20, said neocarzinostatin derivative being represented by the formula:
(SMA)-(NCS)-(SMA)
,wherein (NCS) is a divalent neocarzinostatin residue in which one hydrogen atom is removed from the primary amino group in (a) the alanine residue at the N-terminal of neocarzinostatin and (b) the lysine residue at the 20th position from the N-terminal of neocarzinostatin and (SMA) is a monovalent styrene-maleic acid copolymeric residue, which may be partially half-esterified, and consists of structural units of the formulae

1) a styrene residue of formula

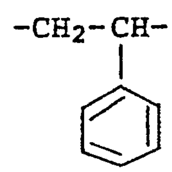

2) a maleic acid residue of formula

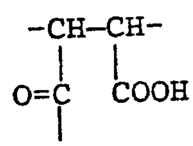

wherein the carbon atom of the carbonyl group is bonded to the neocarzinostatin residue; and
3), a) a maleic acid residue of formula

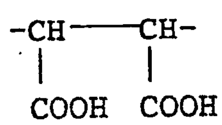

and optionally b) a half-esterified maleic acid residue of formula

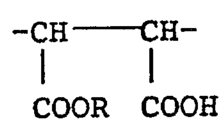

,wherein R is a residue of an alcohol ROH which is an alkanol having 1 to 4 carbon atoms, an

ethylene glycol monoalkyl ether in which the alkyl group has 1 or 2 carbon atoms or a glycerine dialkyl ether wherein the alkyl group has 1 or 2 carbon atoms.

2. A composition according to claim 1, wherein said styrene-maleic acid copolymeric residue is a monovalent partially halfbutylesterified styrene-maleic acid copolymeric residue.

3. A composition according to claim 1 or 2 wherein said medium to long chain fatty acid glyceride comprises a triglyceride of at least one fatty acid having a carbon number of 6 to 20.

4. A composition according to claim 3, wherein said medium to long chain fatty acid glyceride comprises caprilic acid triglyceride and/or capric acid triglyceride.

5. A composition according to any one of the preceding claims which also comprises at least one amphiphilic agent.

6. A composition according to any one of the preceding claims which also comprises a lower alcohol, preferably a $C_1$-$C_4$ alcohol.

7. A method of preparing a composition as claimed in any one of the preceding claims, which comprises adding a lyophilized powder of the neocarzinostatin derivative to the glyceride and uniformly dispersing the powder therein, either or both of the powder and glyceride being optionally premixed.

8. The use in the preparation of a composition for oral administration of a neocarzinostatin derivative as defined in claim 1 and at least one medium to long chain fatty acid glyceride as defined in claim 1.

## Revendications

1. Composition apropriée pour l'administration par voie orale, comprenant un dérivé de néocarzinostatine, et au moins un glycéride d'acide gras ayant une chaîne de moyenne à longue qui est un mono-, di- ou triglycéride d'un acide gras saturé ou non saturé, ayant un nombre de carbones de 6 à 20, ledit dérivé de néocarzinostatine étant représenté par la formule:
(SMA)-(NCS)-(SMA)
dans laquelle (NCS) représente un reste néocarzinostatine bivalent dans lequel un atome d'hydrogène est éliminé du groupe amino primaire dans (a) le reste alanine à la terminaison N de la néocarzinostatine et (b) le reste lysine à la 20ème position à partir de la terminaison N de la néocarzinostatine, et (SMA) représente un reste monovalent copolymère styrène-acide maléique, qui peut etre partiellement semi-estérifié, et se compose d'unités structurales répondant aux formules:

1) un reste styrène de formule

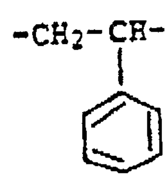

2) un reste acide maléique de formule

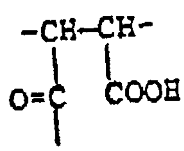

dans laquelle l'atome de carbone du groupe carbonyle est lié au reste néocarzinostatine; et

3), a) un reste d'acide maléique de formule

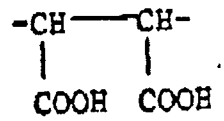

et éventuellement
b) un reste d'acide maléiqué semi-estérifié, de formule

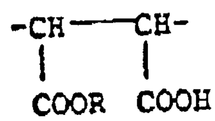

dans laquelle R est un reste d'un alcool ROH qui est un alcanol ayant 1 à 4 atomes de carbone, un éthylèneglycol-monoalkyléther dans lequel le groupe alkyle comporte 1 ou 2 atomes de carbone ou un glycérine-dialkyléther dans lequel le groupe alkyle comporte 1 ou 2 atomes de carbone.

2. Composition selon la revendication 1, dans laquelle ledit reste de copolymère styrène-acide maléique est un reste copolymère styrène-acide maléique monovalent, partiellement semi-butylestérifié.

3. Composition selon la revendication 1 ou 2, dans laquelle ledit g]ycéride d'acide gras, ayant une chaîne de moyenne à longue, comprend un triglycéride d'au moins un acide gras ayant un nombre de carbones de 6 à 20.

4. Composition selon la revendication 3, dans laquelle ledit glycéride d'acide gras, ayant une chaîne de moyenne à longue, comprend le triglycéride de l'acide caprylique et/ou le triglycéride de l'acide caprique.

5. Composition selon l'une quelconque des revendications précédentes, qui comprend également au moins un agent amphiphile.

6. Composition selon l'une quelconque des revendications précédentes, qui comprend également un alcool inférieur, de préférence un alcool en $C_1$ à $C_4$.

7. Procédé de préparation d'une composition telle que revendiquée dans l'une quelconque des revendications précédentes, qui comprend les étapes consistant à ajouter au glycéride une poudré lyophilisée du dérivé de néocarzinostatine et à disperser uniformément la poudre dans celui-ci, la poudre ou le glycéride, ou les deux, étant éventuellcment prémélangés.

8. Utilisation, dans la préparation d'une composition destinée à l'administration par voie orale, d'un dérivé de néocarzinostatine tel que défini à la revendication 1, et d'au moins un glycéride d'acide gras de chaîne moyenne à longue, tel que défini à la revendication 1.

## Ansprüche

1. Zur oralen Verabreichung geeignete Zusammensetzung umfassend ein Neocarzinostatin-Derivat und mindestens ein mittel- bis langkettiges Fettsäureglycerid, das ein Mono-, Di- oder Triglycerid einer gesättigten oder ungesättigten Fettsäure mit einer Kohlenstoffzahl von 6 bis 20 ist, und worin das NeocarzinostatinDerivat dargestellt wird durch die Formel:
(SMA)-(NCS)-(SMA)
worin (NCS) ein zweiwertiger Neocarzinostatin-Rest ist, in dem ein Wasserstoffatom aus der primären Aminogruppe in (a) dem Alaninrest am N-Terminus des Neocarzinostatins und (b) dem Lysinrest an der

20. Position vom N-Terminus des Neocarzinostatins entfernut wurde, und (SMA) ein einwertiger Styrol-Maleinsäure-Copolymer-Rest ist, der teilweise halbverestert sein kann, und aus Struktureinheiten der Formeln

1. eines Styrolrestes der Formel

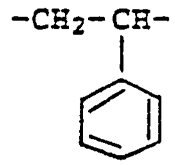

2. eines Maleinsäurerestes der Formel

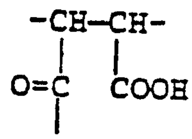

worin das Kohlenstoffatom der Carbonylgruppe an den Neocarzinostatin-Rest gebunden ist; und

3. a) eines Maleinsäurerestes der Formel

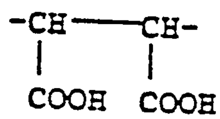

und gegebenenfalls b) eines halbveresterten Maleinsäurerestes der Formel

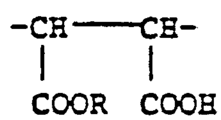

besteht, worin R ein Rest eines Alkohols ROH ist, der ein Alkanol mit 1 bis 4 Kohlenstoffatomen, ein Ethylenglycolmonoalkylether, in dem die Alkylgruppe 1 oder 2 Kohlenstoffatome besitzt, oder ein Glycerindialkylether, worin die Alkylgruppe 1 oder 2 Kohlenstoffatome besitzt, ist.

**2.** Zusammensetzung nach Anspruch 1, dadurch gekennzeichnet, daß der Styrol-Maleinsäure-Copolymer-Rest ein einwertiger teilweise durch Butyl halbveresterter Styrol-Maleinsäure-Copolymer-Rest ist.

**3.** Zusammensetzung nach Anspruch 1 oder 2, dadurch gekennzeichnet, daß das mittel- bis langkettige Fettsäureglycerid ein Triglycerid aus mindestens einer Fettsäure mit einer Kohlenstoffzahl von 6 bis 20 umfaßt.

**4.** Zusammensetzung nach Anspruch 3, dadurch gekennzeichnet, daß das mittel- bis langkettige Fettsäureglycerid Caprylsäuretriglycerid und/oder Caprinsäuretriglycerid umfaßt.

**5.** Zusammensetzung nach einem der vorausgehenden Ansprüche, dadurch gekennzeichnet, daß sie auch mindestens ein amphiphiles Agens umfaßt.

**6.** Zusammensetzung nach einem der vorhergehenden Ansprüche, dadurch gekennzeichnet, daß sie auch einen niedrigen Alkohol, vorzugsweise einen $C_1$-$C_4$-Alkohol, umfaßt.

**7.** Verfahren zur Herstellung einer in einem der vorhergehenden Ansprüche beanspruchten Zusammensetzung, dadurch gekennzeichnet, daß man ein lyophilisiertes Pulver des Neocarzinostatin-Derivats zum

Glycerid zugibt und das Pulver darin gleichmäßig dispergiert, wobei einer oder beide der Bestandteile Pulver und Glycerid gegebenenfalls vorgemischt sind.

8. Verwendung eines wie in Anspruch 1 definierten Neocarzinostatin-Derivats und mindestens eines wie im Anspruch 1 definierten mittel- bis langkettigen Fettsäureglycerids zur Herstellung einer Zusammensetzung für die orale Verabreichung.

FIG_1

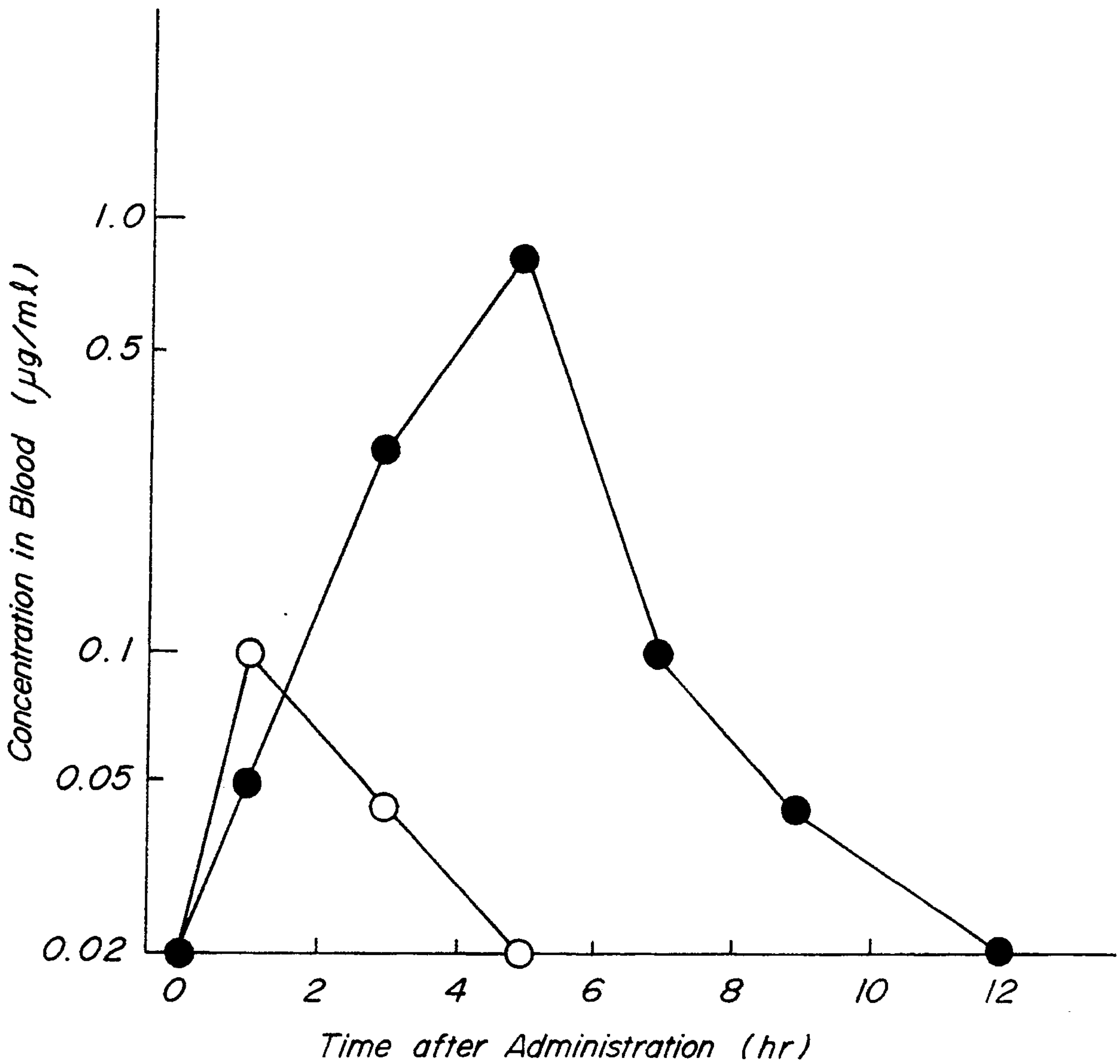
1.0
0.5
0.1
0.05
0.02
Concentration in Blood (μg/mℓ)
0
2
4
6
8
10
12
Time after Administration (hr)

FIG_2

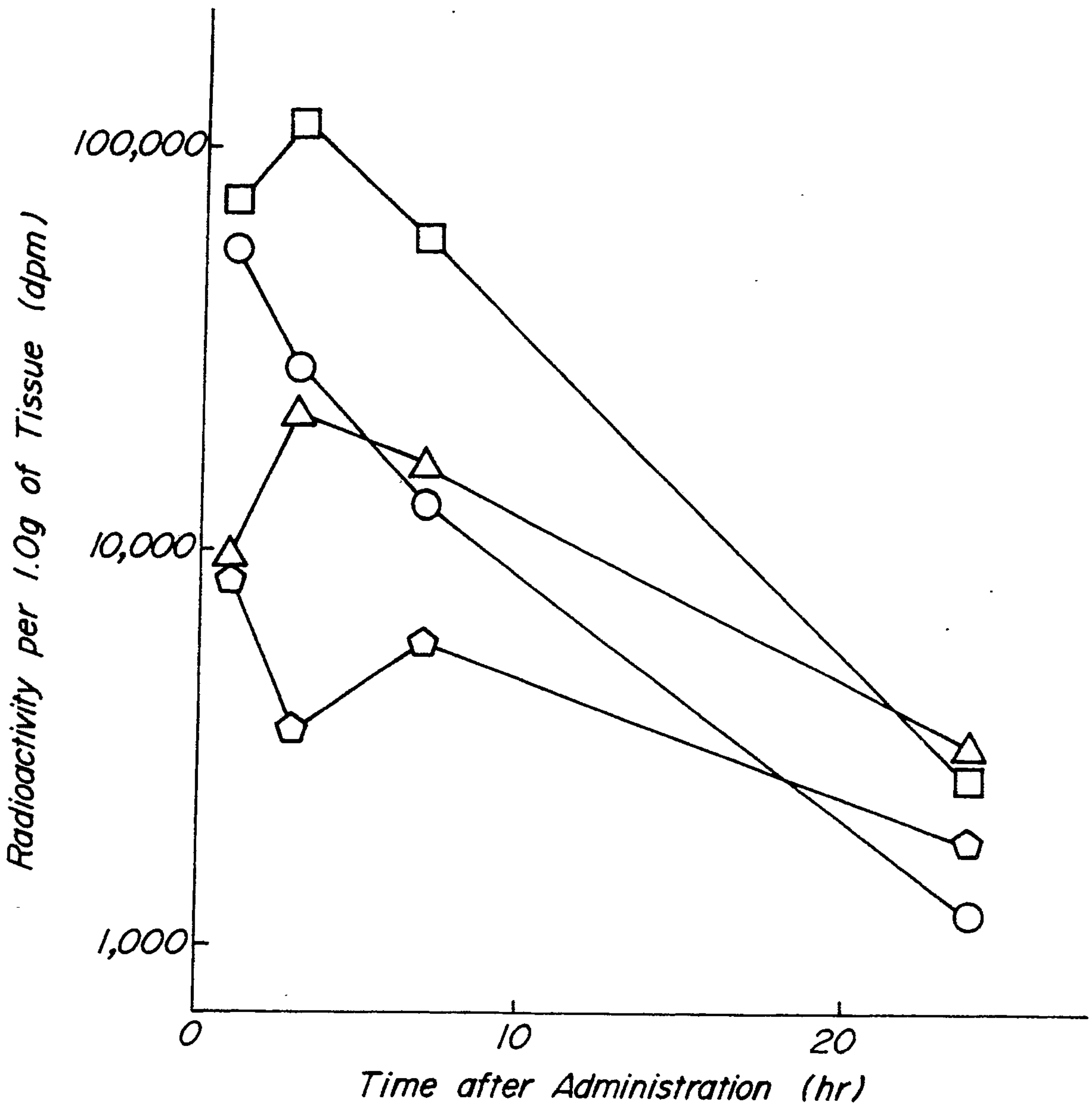
100,000
10,000
1,000
Radioactivity per 1.0g of Tissue (dpm)
0
10
20
Time after Administration (hr)